Europäisches Patentamt

European Patent Office

Office européen des brevets

(11) Numéro de publication: **0 201 387**
**A1**

## (12) DEMANDE DE BREVET EUROPEEN

(21) Numéro de dépôt: **86400803.2**

(51) Int. Cl.⁴: **A 61 K 7/00**, A 45 D 24/00

(22) Date de dépôt: **15.04.86**

(30) Priorité: **17.04.85 FR 8505820**

(43) Date de publication de la demande: **12.11.86**
**Bulletin 86/46**

(84) Etats contractants désignés: **AT BE CH DE FR GB IT LI LU NL SE**

(71) Demandeur: **Chapoton, Christian, 10, rue de la Grande Truanderie, F-75001 Paris (FR)**
Demandeur: **Millet, Jean-Marc, 39, Boulevard de Cimiez, F-06000 Nice (FR)**

(72) Inventeur: **Chapoton, Christian, 10, rue de la Grande Truanderie, F-75001 Paris (FR)**
Inventeur: **Millet, Jean-Marc, 39, Boulevard de Cimiez, F-06000 Nice (FR)**

(74) Mandataire: **Orès, Bernard et al, Cabinet ORES 6, avenue de Messine, F-75008 Paris (FR)**

(54) **Dispositif de traitement de l'épithélium humain externe, son procédé de fabrication et procédé d'utilisation d'un tel dispositif.**

(57) Dispositif pour le traitement de l'épithélium humain externe en particulier de zones sensibles de la peau, comme celles du visage, du cuir chevelu et/ou des cheveux, à l'aide d'un objet destiné à venir au contact de l'épithélium et/ou de ses phanères pour y déposer une substance active.

Ledit objet (10) conformé en tant qu'article de massage de la peau ou en tant qu'instrument de coiffure est, au moins dans ses parties destinées à entrer en contact avec la peau et/ou le cuir chevelu et/ou les cheveux, constitué en une matrice macromoléculaire rigide incorporant au moins une substance active choisie pour son action thérapeutique et/ou cosmétologique et pour être propre à être relarguée vers la surface externe de ladite matrice, d'où elle est éliminée par massage et/ou contact et/ou frottement avec la peau, le cuir chevelu et/ou les cheveux.

0201387

L'invention a pour objet un dispositif de traitement de l'épithélium humain externe, son procédé de fabrication et un procédé d'utilisation d'un tel dispositif.

Elle vise, en particulier, un tel dispositif particulièrement bien adapté au traitement du tissu épithélial constitutif de la peau du visage et/ou du cuir chevelu ainsi que, le cas échéant, de certains phanères comme les cheveux.

L'invention vise également un procédé de fabrication d'un tel dispositif.

L'invention vise aussi un procédé d'utilisation d'un tel dispositif propre à être mis en oeuvre, par exemple, pour le conditionnement des cheveux ou pour transférer et/ou délivrer à l'épiderme, en particulier aux parties sensibles de la peau, comme celle du visage, des substances actives d'hygiène, de cosmétologie ou analogue.

On connaît déjà, par FR-A-2 385 392 un procédé de conditionnement des cheveux suivant lequel on frotte les cheveux avec un article constitué d'un support flexible auquel est associé d'une manière libérable un agent de conditionnement des cheveux, normalement solide, pour transférer une quantité efficace de l'agent de conditionnement à la chevelure. Le support flexible est sous forme d'un papier, d'un tissu ou analogue et exige que l'on mouille les cheveux avant de les frotter avec ledit article. La mise en oeuvre du procédé décrit est ainsi malcommode, à tout le moins impossible à mettre en oeuvre en dehors d'une salle d'eau, de sorte que son utilisation est nécessairement limitée.

On connaît également, par exemple par GB-A-632 544, un peigne de traitement ou de teinture des cheveux réalisé en une masse polymère synthétique chimique inerte, soluble dans l'eau et dans laquelle est incorporée une substance de teinture, de blanchiment ou un parfum soluble dans l'eau. Un tel dispositif présente les mêmes inconvénients que ceux attachés à la mise en oeuvre du procédé évoqué ci-dessus.

Pour s'affranchir de ces inconvénients, on a également proposé des peignes, du type de ceux décrits dans GB-A- 413 767 ou US-A-2 154 822 qui comportent sur leurs dents un produit susceptible d'être déposé sur les cheveux, par exemple à partir d'une fine mèche imprégnée et fixée à certaines des dents du peigne, dans le brevet britannique, ou à partir d'une masse cireuse placée sur les dents, dans le brevet américain.

A côté de ces dispositifs connus on a déjà proposé, dans un tout autre domaine, un procédé de lutte contre les ectoparasites d'un animal selon lequel on brosse ou peigne son pelage à l'aide d'un article qui présente des parties en saillies dont certaines au moins sont en un matériau polymère comprenant un pesticide susceptible d'affleurer à la surface de l'article, EP-A-0 122 605. L'insecticide déposé sur les poils est inhalé ou ingéré par les tiques, puces ou autres ectoparasites qui se déplacent dans le pelage de l'animal jusqu'à ce que soit atteinte la dose létale qui les fait disparaître. Le dispositif n'est pas destiné à amener au contact de la peau fragile de l'animal une substance active mais à placer dans le pelage dense, qui comprend trois types de poils, duvet, barbe et jarre, l'insecticide souhaité.

Par rapport à cet état de la technique très hétérogène, puisqu'il englobe des objets aussi divers que des peignes selon le Brevet britannique, des serviettes selon le Brevet français, ou des brosses pour chiens selon la Demande européenne, les Demandeurs se sont posés le problème de fournir un dispositif de traitement de l'épithélium humain externe, en particulier de la peau du visage, du cuir chevelu et des cheveux qui permet d'agir efficacement contre certains troubles pathologiques de cet épithélium, par exemple l'apparition de rides, la perte de cheveux, l'apparition de pellicules, le psoriasis, la séborrhée et d'autres phénomènes liés au vieillissement, ainsi que de traitement de cosmétologie, notamment du visage et de la chevelure.

En outre, les Demandeurs ont cherché à résoudre le problème posé à l'aide d'un dispositif dont l'emploi

soit aussi simple que possible, qui ne fasse appelle qu'à ce seul dispositif, qui ne nécessite pas de dosage, qui puisse être mis en oeuvre par des gestes simples et usuels, et qui, complémentairement, ne se présente pas sous forme liquide ou visqueuse comme certaines crèmes ou lotions connues de sorte que l'application et le transfert sur l'épithélium à traiter de substance(s) active(s) peut être envisagé par des applications répétées de faible ou très faible quantité chacune.

Pour tenter d'apporter une réponse au problème posé, les Demandeurs ont pensé avoir recours aux systèmes polymériques matriciels à libération lente développés depuis de nombreuses années pour l'administration par voie transdermique de substances médicamenteuses. Les essais entrepris dans cette voie se sont cependant révélés infructueux, les dispositifs connus prévus pour une application permanente sur l'épiderme étant totalement inappropriés pour une utilisation répétée, mais intermittente, sur la peau du visage ou le cuir chevelu. En effet, alors que l'on exige des dispositifs transdermiques d'application de substance(s) médicamenteuse(s) qu'ils soient présentés sous des formes souples, comme des bandes ou des bracelets susceptibles de se déformer avec les mouvements de leurs utilisateurs, on demande au contraire aux dispositifs de peigne, brosse ou analogue, qu'ils soient résistants pour une utilisation prolongée et rigides pour supporter les efforts relativement importants auxquels ils sont soumis lorsqu'ils sont employés dans des chevelures humaines. Il en résulte aussi des différences de méthodes de fabrication. En effet, si l'on cherche à accroître la rigidité d'une matrice polymère, par exemple en réduisant la quantité de plastifiant qui lui est généralement associée dans les dispositifs connus, le moulage ne peut plus être réalisé dans les conditions connues pour les dispositifs de transfert de substances médicamenteuses évoqués ci-dessus; la température de fabrication doit être accrue, mais si l'on atteint celles mises en oeuvre dans la fabrication des brosses ou peignes usuels en matière plastique, celles-ci sont très notablement supérieures à la température de destruction des principes actifs qui pourraient être utilisés.

De manière surprenante, les Demandeurs ont maintenant établi que par un choix approprié de polymères destinés à former la matrice et de la ou des substance(s) active(s), ainsi que d'adjuvants choisis de manière appropriée, il est possible de fournir un dispositif de traitement de l'épithélium externe humain, en particulier de certaines zones sensibles de la peau, comme celles du visage, du cuir chevelu et/ou des cheveux qui satisfasse aux conditions énoncées ci-dessus.

Un dispositif selon l'invention, pour le traitement de l'épithélium humain externe en particulier de zones sensibles de la peau, comme celles du visage, du cuir chevelu et/ou des cheveux à l'aide d'un objet destiné à venir au contact de l'épithélium et/ou de ses phanères pour y déposer une substance active, est caractérisé en ce que ledit objet, conformé en tant qu'article de massage de la peau ou en tant qu'instrument de coiffure est, au moins dans ses parties destinées à entrer en contact avec la peau et/ou le cuir chevelu et/ou les cheveux, constitué en une matrice macromoléculaire rigide incorporant au moins une substance active choisie pour son action thérapeutique et/ou cosmétologique et pour être propre à être relarguée vers la surface externe de ladite matrice , d'où elle est éliminé par massage et/ou contact et/ou frottement avec la peau, le cuir chevelu et/ou les cheveux.

Dans une forme de réalisation, l'article de massage ou l'instrument de coiffure conformé suivant un peigne, une brosse, une barrette, un bigoudi, un rouleau, une broche, une pince ou analogue comprend une armature rigide sur laquelle est fixée la matrice macromoléculaire incorporant la ou les substances actives.

5

Dans une autre réalisation, les zones destinées à entrer en contact avec la peau et/ou le cuir chevelu et/ou les cheveux et qui sont réalisées en ladite matrice macromoléculaire armée ou non armée forment un ensemble fixé de manière amovible ou non sur un manche, poignée de préhension ou analogue en un matériau différent de celui de la matrice macromoléculaire.

Selon l'invention, cette dernière est choisie pour contenir un ou des agents à action thérapeutique et/ou cosmétologique actifs dans le traitement de la peau, en particulier le traitement des phénomènes liés au vieillissement comme les rides du visage.

Dans une autre forme de réalisation, la ou les substances actives est(sont) choisie(s) parmi celles contenant un ou des agents actifs dans le traitement de l'hyperséborrhée, du psoriasis, de la chute des cheveux, de la disparition des pellicules, ou de l'aide à la repousse des cheveux.

Dans encore une autre forme de réalisation, la ou les substances actives est(sont) choisie(s) parmi celles contenant un ou des agents à action cosmétique pour faciliter le coiffage, le démêlage, le conditionnement, le nettoyage, la modification de la texture, de la couleur, et/ou de l'aspect des cheveux.

Selon une autre caractéristique de l'invention la ou les substances actives a(ont) un rôle de plastifiant de la matrice macromoléculaire.

Dans une forme de réalisation la substance active contient une ou des vitamines et/ou des acides gras insaturés.

Dans une autre forme de réalisation, la substance active contient de 1 à 25 % d'extraits végétaux et/ou animaux et/ou d'essences végétales.

Dans une forme de réalisation préférée, la substance active contient un ou des ingrédients favorisant le passage transcutané des agents actifs.

6

Dans une forme de réalisation avantageuse, la matrice contient de 3 à 25 % de pyrithione ou de ses dérivés en tant qu'agent actif.

Dans encore une autre forme de réalisation la matrice contient de 1 à 25 % de coal-tar ou de ses dérivés en tant qu'agent actif.

Un dispositif selon l'invention permet ainsi de répartir un ou des agents actifs sur la peau du visage et/ou le cuir chevelu et éventuellement les cheveux avec des gestes simples, sans utilisation d'autres produits que le dispositif, sans avoir à effectuer de dosage et sans avoir à utiliser de crème, lotion ou analogue.

Il permet également d'accroître la fréquence de traitement, facteur essentiel du résultat thérapeutique, tout en éliminant les effets rebonds connus avec d'autres moyens d'application, c'est-à-dire, par exemple, la réapparition de pellicules en quantité plus importante quelques jours après un shampooing même si elles ont disparues partiellement immédiatement après le shampooing.

Lorsque le dispositif est sous forme de brosse la substance active est répartie à chaque massage sur la peau ou à chaque coiffage (brosse ou peigne) dans la profondeur de la chevelure et au contact du cuir chevelu en assurant un massage de ce dernier.

Parmi les nombreuses réalisations du dispositif selon l'invention, une forme préférée est celle d'un stylo dont au moins l'extrémité, aplatie ou dentelée, est réalisée dans la matrice active pour être utilisé en tant qu'applicateur de substance active par massage de la peau et/ou du cuir chevelu.

Dans le cas des autres instruments de coiffure, la substance active relarguée puis éliminée par frottement (surtout avec les cheveux) migre de proche en proche dans la chevelure et atteint le cuir chevelu, se maintenant grâce à la fréquence usuelle de l'usage de ces instruments en quantité suffisante pour être active.

Selon une autre caractéristique importante de la présente invention la substance active ne laisse pas la peau du visage, le cuir chevelu et/ou les cheveux graisseux ou poudreux.

De bons résultats ont été obtenus à l'aide de dispositifs selon l'invention dans le traitement de la peau du visage et/ou du cuir chevelu et/ou des cheveux nonobstant le caractère relativement acide et l'importante couverture lipidique de cet épithélium externe humain, avec des quantités de substances actives libérées sur leurs sites d'action notablement plus faibles que celles mises en oeuvre dans les méthodes d'application usuelles.

L'invention a également pour objet un procédé de fabrication d'un dispositif tel que décrit ci-dessus, caractérisé en ce que l'on prépare une poudre sèche par mélange à une température d'environ 60° d'un ou des polymères constitutifs de la matrice avec un ou des agents actifs préalablement micronisé(s) lorsqu'il(s) est(sont) sous forme solide ou microsolubilisé(s) dans un mélange de plastifiant et d'adjuvants, en ce qu'on laisse refroidir ladite poudre à laquelle sont alors ajoutés des ingrédients, comme des parfums ou analogues, et en ce que le dispositif est mis en forme par moulage ou extrusion à une température comprise entre 80 et 140°C dans un moule de forme appropriée.

Dans une variante de mise en oeuvre du procédé certains agents actifs sont solubilisés dans un solvant organique préalablement au mélange avec le ou les plastifiant(s), le ou les adjuvant(s) et la matrice macromoléculaire pour améliorer l'homogénéité et favoriser le relargage de la substance active à partir du dispositif fini.

La température relativement peu élevée de moulage ou d'extrusion, qui permet de ne pas dégrader la ou les substance(s) active(s), est permise par un choix approprié des formulations de la matrice et grâce à une vitesse plus lente que normale de l'extrusion ou du moulage.

Dans le cas de moulage par injection la matière reste plus longtemps dans le pot avant injection afin d'être portée à l'état fondu à une température homogène avant d'être refoulée dans les cavités du moule ayant la configuration des parties actives comme des dents de peignes, de brosses, des instruments de coiffure ou des parties seulement de ces dispositifs comme par exemple les dents pour un peigne.

Dans une forme de réalisation du procédé, la mise en forme par moulage ou extrusion ne vise que les parties actives du dispositif qui sont ensuite fixées à un support éventuel comme un manche ou une poignée en le même matériau que celui constitutif de la matrice,mais sans substance active,ou en un matériau différent.

Dans une variante on réalise, simultanément, la mise en forme des parties actives et non actives.

Pour l'obtention de la rigidité requise du dispositif selon l'invention on prévoit de limiter la quantité de plastifiant que comprend la matrice à des valeurs comprises entre 0 et 25 % en poids de la matrice dans le cas d'agents actifs ayant des propriétés plastifiantes, le ou les polymères représentant environ 45 à 90 % en poids de la matrice, suivant la substance active mise en oeuvre.

Dans une variante d'exécution, le moulage ou l'extrusion des parties actives du dispositif a lieu sur une armature en matière plastique ou en un autre matériau approprié.

L'étape du procédé selon laquelle la substance est micronisée lorsqu'elle est sous forme solide, ou micro-solubilisée lorsqu'elle est sous forme liquide préalablement à son mélange aux ingrédients constitutifs de la matrice permet d'assurer un relargage satisfaisant de la substance active vers la surface de la matrice, eu égard à l'efficacité du ou des agents actifs composant la substance active, laquelle est incorporée dans la matrice à raison de 2 à 25 % en poids.

L'invention a également pour objet un procédé d'utilisation d'un dispositif tel que décrit ci-dessus pour l'application sur l'épithélium externe humain, en particulier les zones sensibles de la peau, comme celle du visage, le cuir chevelu et/ou les cheveux, d'un ou de principe(s) actif(s) contre le vieillissement et/ou les états pathologiques dudit épithélium et/ou pour le conditionnement . de ses phanères, caractérisé en ce que ledit dépôt résulte de massages, contacts et/ou frottements répétés, mais espacés dans le temps, sur et/ou avec les zones à traiter d'un dispositif constitué par une matrice macromoléculaire dans laquelle est incorporée au moins une substance active propre à être relarguée vers la surface et dont elle est éliminée par lesdits massages, contacts ou frottements.

Le procédé selon l'invention peut être mis en oeuvre pour traiter des troubles de la peau et/ou du cuir chevelu et des cheveux incluant les phénomènes liés au vieillissement : rides du visage et chute des cheveux, en particulier les pellicules.

Dans le cas de lutte contre les rides du visage, ou d'autres zones sensibles de la peau, l'apport de la substance active est associé à un massage simultané à la libération de substance active qu'il favorise. Tout risque de surdosage de ladite substance est exclu étant donné que la libération lente hors de la matrice de ladite substance exige un certain temps pour que la concentration à la périphérie et à la surface de ladite matrice revienne à son niveau d'avant le début du massage.

Dans une telle utilisation, la substance active comprend avantageusement des éléments nutritifs tels que des vitamines, des extraits biologiques végétaux ou animaux comme des hormones, des huiles essentielles, des extraits tissulaires ou des dérivés des substances constituant la matrice inter-cellulaire du derme.

Lorsque le procédé vise le traitement du cuir chevelu et des cheveux, il est principalement destiné à combattre les pellicules, la séborrhée, le psoriasis, la chute des cheveux ou aider à leur repousse.

Dans ce cas, la substance active comprend avantageusement des goudrons ou des dérivés de goudrons, comme le coal-tar, des huiles minérales ou végétales ou encore des pyrithiones et leurs dérivés, des acides gras insaturés, etc ...

Lorsque le procédé est mis en oeuvre pour le conditionnement des cheveux dans un but cosmétique ou d'hygiène : coiffage, démêlage, fixation, nettoyage, modification de la texture, de la couleur ou de l'aspect des cheveux, la substance active contient avantageusement un agent absorbant les graisses comme de la poudre d'iris ou de lycopode, un agent détergent ou des oligomères de silicone, des dérivés d'acides gras, des composés d'ammonium quaternaires, etc...

De façon avantageuse l'invention envisage également de combiner des substances actives de l'un et l'autre type, une telle combinaison ayant un effet synergique pour l'obtention d'un dispositif à effet à la fois thérapeutique et cosmétique.

L'invention sera bien comprise par la description qui suit, faite à titre d'exemple et en référence au dessin annexé dans lequel :

- la figure 1 est une vue schématique de formes de réalisation d'un dispositif selon l'invention ;

- la figure 2 est une vue schématique en perspective d'un autre dispositif selon l'invention ;

- la figure 3 est une vue schématique, également en perspective, d'encore un autre dispositif.

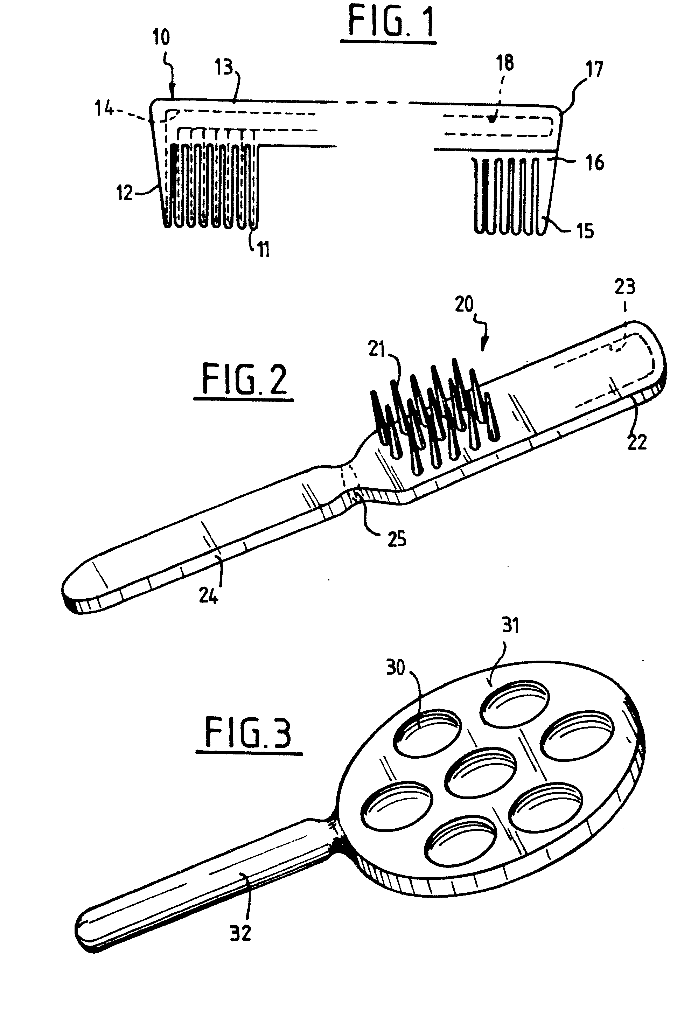

On se réfère d'abord à la figure 1 qui montre schématiquement diverses formes de réalisation d'un dispositif selon l'invention.

Dans une première réalisation, le dispositif

11

est sous forme d'un peigne à dents 11 constitué d'une matrice macromoléculaire dans laquelle est incorporée une substance active propre à être relarguée vers la surface externe 12 des dents lorsque le peigne est passé dans la chevelure.

Dans la réalisation illustrée sur la partie gauche du dessin, les dents 11 et le corps 13 sont mis en forme sur une armature 14, montrée en trait mixte, et qui comprend elle aussi un corps et des dents en un matériau de plus grande rigidité que celui constitutif de la matrice macromoléculaire, tandis que sur la partie droite du dessin, les dents 15 et leur embase 16 sont conformés suivant un ensemble sans armature, intégralement réalisé en ladite matrice macromoléculaire et solidarisé ensuite avec un corps 17 avec ou sans armature 18. Cette dernière réalisation permet de limiter l'utilisation de substance active aux seules parties comme les dents 15 destinées à venir en contact avec la chevelure, et/ou le cuir chevelu, permettant ainsi de réduire les coûts mais aussi d'éviter le dépôt de substance active sur la main de l'utilisateur qui n'a pas à être traitée.

Dans la forme de réalisation montrée sur la figure 2, le dispositif est conformé suivant une brosse 20 à dents 21 qui forme avec le corps 22 un ensemble en une matrice macromoléculaire incorporant au moins une substance active propre à être relarguée vers la surface des dents et dont elle est éliminée par frottement. Dans cette forme de réalisation, également, le corps 22 est avantageusement moulé sur une armature 23 destinée à donner à l'ensemble la rigidité requise et le corps 22 est relié à un manche ou poignée 24 qui peut être d'un bloc avec le corps 22 ou qui peut être relié à celui-ci de manière amovible. Quel que soit le mode de liaison, et dans le cas où la poignée 24 est également en un matériau polymère, on prévoit de réaliser la zone de liaison 25 entre le corps 22 et la poignée ou manche 24 avec une section aussi faible que possible, pour éviter les phénomènes d'inter-diffusion entre la matrice macromoléculaire à substance active incorporée et le matériau constitutif du manche ou poignée.

Dans la forme de réalisation montrée sur la figure 3, le dispositif est conformé en tant qu'article de massage avec des billes 30 en une matrice macromoléculaire incorporant une substance active propre à en être relarguée vers la surface et dont elle est éliminée par contact avec les zones de la peau sur laquelle est appliqué l'article. Dans cette réalisation, les billes 30 sont montées à rotation libre dans un corps 31 solidaire d'une poignée ou manche 32, le corps 31 et/ou la poignée 32 étant avantageusement en des matériaux autres que ceux constitutifs de la matrice macromoléculaire, ou en le même matériau mais sans substance active.

Quel que soit le mode de réalisation du dispositif selon l'invention, et son procédé de fabrication, on prévoit de le conditionner dans un moyen d'emballage étanche, dont il est sorti lors de la première utilisation et à partir de laquelle il peut ensuite être utilisé pour une durée de l'ordre de 4 semaines à quelques mois en fonction de la substance active relarguée, et de la formulation de la matrice.

Cette dernière est choisie en fonction de la substance active à y incorporer et est de préférence formée par un ou un mélange de polymères synthétiques organiques, un ou un mélange de polymères naturels (biopolymères), de polymères inorganiques, de polymères synthétiques ou élastomères synthétiques.

Les exemples de substances macromoléculaires utilisables selon l'invention sont des polyoléfines (polyéthylène, polypropylène, copolymères d'éthylène et propylène, par exemple), des polyacrylates (polymères et copolymères d'acrylate de méthyle, d'acrylate d'éthyle, de métacrylate de méthyle, de métacrylate d'éthyle, par exemple), des polymères de composés vinyliques (du polystyrène ou du divinyl-benzène polymérisé, par exemple), des esters de polyvinyle, des halogénures de polyvinyle (du chlorure de polyvinyle), des acétates polyvinyliques (du butyral polyvinylique, par exemple), des composés de vinylidène (du chlorure de poly-vinylidène, par exemple), des copolymères d'éthylène et d'acétate de vinyle, des élastomères synthétiques et naturels (du caoutchouc obtenu à partir d'hevea brasiliensis, du cis-4-polyisoprène, du polybutadiène ou du caoutchouc SBR ou SBS ou SEPS ou CEPC ou CEBC), des résines urées-formaldéhyde et mélamine-formaldéhyde, des résines époxy (des polymères d'éthers polyglycidiques de phénols polyhydriques, par exemple), des matières plastiques cellulosiques (l'acétate, le phtalate de cellulose, le butyrate de cellulose, le nitrate de cellulose, par exemple), et des polyuréthanes.

On peut également utiliser d'autres résines naturelles comme les paraffines, ou d'autres polymères synthétiques comme par exemple les polyvinyl alcools, le poly-acrylamide, les polyethers, les polyamides, les polyurées, le polyacrylonitrile, le méthacrylate de polyméthyle.

Sans que cela soit toujours nécessaire, la matrice macromoléculaire comporte en général un ou (des) plastifiants(s).

On peut utiliser pour la mise en oeuvre de l'invention, des plastifiants usuels et par exemple des phta-lates comme le dibutylphtalate, le dioctylphtalate, des sébaçates, par exemple le sébaçate de dipentyle, de dibenzyle, des adipates, par exemple, le dioctyladipate, dibutyladipate, des polyphénols hydrogénés, des hydrocarbures aromatiques alcoylés, des plastifiants du type polyphénols hydrogénés, des hydrocarbures aromatiques alcoylés, des plastifiants du type polyester, comme des polyesters de polyols, par exemple l'hexanediol, ou du type acide carboxylique, comme l'acide sébacique ou adipique.

Dans le cas de substances actives telles que définies ci-après, incorporant des agents actifs ayant des propriétés plastifiantes, ceux-ci sont utilisés à la fois comme agent actif et plastifiant, seuls ou associés à un plastifiant usuel, ce dernier étant alors en quantité moindre que d'usage. De ce fait, on peut incorporer dans le dispositif une plus grande quantité de substance active et conserver une rigidité suffisante.

La matrice comprend également, le cas échéant, d'autres adjuvants tels que des stabilisants, antioxydants, charges, lubrifiants, agents de démoulage, agents antistatiques, colorants ou parfums.

La substance active est choisie en fonction de ses propriétés pour le traitement de l'épithélium externe et/ou de ses phanères, par exemple, la peau du visage et/ou du cuir chevelu, et/ou des cheveux, et pour son aptitude à être incorporée à la matrice, à être relarguée et à en être éliminée par massage et/ou contact et/ou frottement avec la peau du visage, les cheveux et/ou le cuir chevelu .

La substance active est avantageusement choisie parmi celles ayant une ou plusieurs des activités suivantes : antifongique, antibactérienne, antiseptique, anti-séborrhéique, kératolytique, anti-inflammatoire, anti-psoriasis, anti- prurigineuse, cicatrisante, antipelliculaire, nutritive, hor- monale ou favorisant la croissance du cheveu.

D'autres substances actives sont choisies pour leurs propriétés cosmétiques ou nettoyantes.

Dans un premier mode particulier de réalisation de l'invention, la substance active confère au dispositif une action anti-pelliculaire, ou anti-séborrhéique et/ou anti- psoriasis.

Elle est alors choisie parmi des goudrons ou des dérivés des goudrons, dont les propriétés plastifiantes ont été mises en évidence dans le cadre de l'invention, comme les goudrons de houille (coal-tar), sous forme d'extrait, de solution de distillats, et ses dérivés : crésol, phénol, phénolate de sodium, du chloroxylénol, alkylphénol polyoxyéthylène, éthohéxadiol ou les goudrons de bois (bouleau, pin, panama, genevrier...) leurs dérivés comme les saponines, mais aussi des huiles minérales ou extraits ou huiles végétales comme l'huile de cade, l'huile essentielle de bouleau, l'huile de lin, de pétrole, l'ichthammol, des extraits d'ortie, de racine de bardane, pariétaire, jamborandi, capucine, saule, Swertia Jopanica.

Il a été constaté de manière surprenante que l'incorporation de ces agents dans la matrice, malgré leur complexité chimique (en particulier celle du coal-tar) au niveau de la multitude de molécules actives différentes, permettait un relargage satisfaisant en ce qui concerne l'efficacité clinique par rapport à une application traditionnelle des mêmes agents actifs. D'autres substances actives utilisées seules ou en association sont choisies parmi les essences végétales comme le thymol, le menthol, le terpinéol, l'eucalyptol, aux propriétés antibactériennes ou anti-inflammatoires, en particulier le alpha-bisabolol.

D'autres substances actives sont constituées par les pyrithiones et leurs dérivés, par exemple le pyrithione zinc, le pyrion disulfide (ND), ou le ciclopirox et ses dérivés, par exemple le ciclopirox olamine, la piroctone olamine ou des dérivés de l'alpha ou béta-thujaplicine, en particulier des sels de cuivre ou d'autres agents à activité antifongique tels que : enilconazol, clotrinazole, miconazole, econazol, isoconazol, tolnaftate, pimaricine, nistatine, ketonazol.

Parmi d'autres substances actives ayant donné de bons résultats, il faut encore citer l'acide salicylique et ses dérivés alcalins ou le méthylsalicylate ainsi que les sulfures de sélénium, les sulfures, les dérivés soufrés, le soufre, le sulfate de zinc, le résorcinol, le captan, l'acide undécylénique et ses dérivés.

L'invention peut également être mise en oeuvre dans un dispositif comportant en tant que substance active seule ou en mélange, l'acide benzoïque et/ou l'acide borique et ses dérivés comme le borate de sodium, ainsi que la chlorhéxidine et l'hexamidine.

Le camphre, connu comme plastifiant à des concentrations très faibles pour les esters et éthers de cellulose, peut être incorporé à des concentrations de l'ordre de 5 à 15 %. Afin d'améliorer ses propriétés de migration dans la matrice il est solubilisé dans un solvant organique avant incorporation.

On peut également utiliser le di-oxyanthranol et/ou le parahydroxybenzoate et/ou la benzocaïne (éthylaminobenzoate).

Dans un autre mode de réalisation de l'invention la substance active a une activité dans la prévention des troubles dus au vieillissement de la peau et/ou du cuir chevelu, en agissant contre les rides et/ou la chute des cheveux ou en favorisant leur repousse.

Dans ce cas, la substance active est choisie parmi les hormones de synthèse, végétales ou animales, comme l'oestradiol, la progestérone, le cynosterone acétate et/ou l'hydrocortisone et leur dérivés et/ou des vitamines des groupes A,B, C, D, E, F, H, PP et de leurs dérivés, c'est-à-dire l'acide panthothénique et ses dérivés sous forme de sels de calcium et de sodium, l'acide nicotinique et ses dérivés sous forme de nicotinate d'éthyle ou de méthyle, l'acide paraaminobenzoïque, la pyridoxine et son chlorhydrate , l'acide rétinoïque, la riboflavine $(B_2)$, l'acide folique $(B_6)$.

De bons résultats ont été obtenus avec certaines vitamines comme les vitamines $B_6$, H, PP, C, pantothénol connues pour l'importance de leur action au niveau des épithélium et phanères mais qu'il n'avait pas été possible d'utiliser de manière satisfaisante jusqu'à présent en raison de leur instabilité fréquente et de leur stabilité sélective mais que le procédé selon l'invention permet d'incorporer, de façon surprenante, à une matrice macromoléculaire, telle que décrite ci-dessus. La vitamine F peut être utilisée non seulement pour ses propriétés plastifiantes, mais encore comme solvant et agent favorisant le relargage pour les vitamines A et E et leurs dérivés. La vitamine E a le double intérêt d'avoir une activité propre au niveau de la chevelure et une activité anti-oxydante pour les vitamines A et F lors de la mise en oeuvre du procédé de fabrication.

D'autres agents actifs dans la prévention des troubles dus au vieillissement de la peau et/ou du cuir chevelu utilisables selon l'invention, sont des extraits biologiques végétaux et animaux comme les huiles essentielles, la farine d'avoine colloïdale, l'huile de germe de blé, l'huile d'olive, des extraits de ginseng , des extraits placentaires, des extraits de liquide amniotique, des extraits de chignon de cheval, des extraits tissulaires, de la lécithine ou des dérivés métaboliques tels que l'allantoïne de la propolis.

Parmi les dérivés biologiques d'origine animale, des dérivés des substances constituant la matrice inter-cellulaire du derme sont particulièrement intéressants dans les soins de la peau, du cuir chevelu et de la chevelure. Il s'agit en particulier de fractions de macromolécules : kératine, collagène, élastine, mucopolysaccharides, protéo-glycannes et glycoprotéines de structures ainsi que des phospholipides et lipoamino acides. Des hydrolysats des macropro-téines sont surtout constitués d'acides aminés et de certains sucres ou dérivés : hydroxyproline, desmosine, N-acétyl-glucosamine, acide glucuronique, glucosamine, tryptophane, cystéine par exemple. Parmi ces substances : la DL-méthionine et la S-carboxyméthylcystéine et l'acide glucuronique sont particulièrement intéressantes dans le soin du cuir chevelu ainsi que la cystine et le sébacate de méthylcystéine.

Comme agent actif pour la repousse des cheveux, sont particulièrement efficaces le dichlorophène et diétha-noldodécanamide, le chlorure de caproyle, le chlorhydrate de diphénhydramine, l'hydrochlorure de tétracaïne et le mino-xidile et leurs dérivés , le dinitrochlorobenzène.

Dans un autre mode de réalisation de l'invention, la substance active est choisie surtout pour ses propriétés cosmétiques et d'hygiène parmi les oligomères de silicones : oligomère de polydiméthylsiloxanes, de copolymères silicone-glycol, de cyclométhicones, de amidométhicone et dérivés d'acides gras comme leurs esters ou polyesters, des composés d'ammonium quaternaire comme leurs esters, des alcanolamides, des polyglycols esters, des alcools gras ou à poids moléculaire élevé et leurs dérivés comme des poly-glycoléthers d'alcool gras, ou des dérivés bétaïniques comme le amidoéthylbetaïne de coco ou le oléamidopropylbétaïne de coco.

Certains de ces agents actifs sont utilisés dans le domaine des plastiques en tant que lubrifiants internes (alcools gras ou à poids moléculaire élevé et leurs dérivés) et externes (acidesgras et dérivés), agents de démoulage (silicones) agents antistatiques (dérivés d'ammonium quaternaire et dérivés bétaïniques) par exemple, et à des concentrations faibles (de l'ordre de 2 %).

Dans un but d'hygiène ou de nettoyage on utilise comme agent actif de la poudre d'iris ou de lycopode et/ou tout autre agent absorbant les graisses et/ou à action détergente. Lorsque l'agent actif est une poudre absorbant les graisses, sa concentration dans la matrice est comprise entre 15 et 30 %.

Selon l'invention, les dispositifs montrés sur les figures 1 à 3, mais dont les formes peuvent être différentes de celles illustrées, par exemple, être constituées par des instruments de coiffure, comme des barrettes, bigoudis, rouleaux, broches, pinces, etc... sont conformés en totalité ou dans leurs parties actives seulement, qu'il s'agisse des dents des peignes ou brosses ou des billes ou analogues des articles de massage, à partir des ingrédients mentionnés ci-dessus par un procédé selon lequel on prépare une poudre sèche par mélange à une température d'environ 60° d'un ou des polymères constitutifs de la matrice avec un ou des agents actifs préalablement micronisés lorsqu'ils sont sous forme solide ou microsolubilisés dans un mélange de plastifiant et d'adjuvants, on laisse refroidir

19

ladite poudre à laquelle sont alors ajoutés des ingrédients, comme des parfums ou analogues, et le dispositif est mis en forme par moulage ou extrusion à une température comprise entre 80 et 140°C dans un moule de forme appropriée.

Les exemples qui suivent illustrent d'autres caractéristiques, avantages et particularités de l'invention.

I - DISPOSITIFS DE TRAITEMENT ANTIPELLICULAIRE, ANTISEBORRHEIQUE ET ANTIPSORIASIS :

On prépare les compositions suivantes en parties en poids :

| EXEMPLES : | n° 1 | n° 2 | n° 3 | n° 4 |
|---|---|---|---|---|
| Pyrithione Zinc .... (micronisée puis microsolubilisée dans le DBP) | 16 % | | | |
| Octopirox ....... (micronisé puis microsolubilisé dans le dioctyladipate DOA) | | 12 % | | |
| Coal-tar saponiné ... | | | 19 % | 7 % |
| Essences végétales .. . | 4 % | 4 % | | 2,5 % |
| Huile de soja époxydée. | 4 % | 2,5 % | 2 % | 2 % |
| Stéarate de calcium .. | 2,5 % | 2,5 % | 2 % | 1,5 % |
| Dioctyladipate .. ... | 20 % | | 6 % | |
| Dibutyl phtalate ... | | 15 % | | 11 % |
| Silice ........ | 4 % | 6 % | | |
| Butadiène acrylonitrile | | | 2 % | |
| P.V.C. ........ | 49,5 % | 58 % | 69 % | 22,5 % |
| Polyéthylène ..... | | | | 53,5 % |

Les compositions ci-dessus sont ensuite mises en forme par moulage à une température de l'ordre de 95° C.

20

| EXEMPLES N° 5 | | N° 6 | |
|---|---|---|---|
| Acide salicylique . . . (micronisé) | 15 % | Coal-tar (extrait) . . | 14 % |
| | | Huile minérale . . . . | 10 % |
| Propyléneglycol . . . . | 5 % | Essences végétales . . | 1 % |
| D.O.A. . . . . . . | 20 % | Oxyde de Zinc . . . . . | 5 % |
| Acide myristoléique . . | 1 % | Acide stéarique . . . . | 3 % |
| Millicarb . . . . . . . | 8 % | Soufre . . . . . . . . . | 2,5 % |
| Huile de soja époxydée. | 2 % | C.B.S. . . . . . . . . . | 0,8 % |
| Stéarate de calcium . . | 2,5 % | Argile . . . . . . . . | 20 % |
| P.V.C. . . . . . . . . | 46,5 % | Caoutchouc SBT . . . . | 13,7 % |

La composition de l'Exemple N° 5 est mise en forme à température relativement basse, de l'ordre de 80-85°C et cela pour tenir compte du fait que l'acide salicylique qui se sublime à 76°C se dégrade rapidement par chauffage à la pression atmosphérique.

La composition de l'Exemple N° 6 est mise en forme, quant à elle, par un traitement de vulcanisation de 12 minutes à environ 140°C.

EXEMPLES : N° 7

Des compositions comparables à celles de l'Exemple N° 5 ont été préparées en utilisant du sulfure de sélénium et du résorcinol et lesdites compositions ont ensuite été mises en forme comme indiqué pour cet Exemple.

N° 8

Des compositions comparables à celles de l'Exemple N° 1 ont été préparées en utilisant de l'acide undécylénique, de l'undécylénate de zinc et/ou de sodium.

Les compositions ont ensuite été mises en forme comme décrit ci-dessus en référence à l'Exemple N° 1.

N° 9

Une composition comparable à celle de l'Exemple N° 2 a été préparée en utilisant de l'éthohexadiol. La composition a ensuite été mise en forme comme indiqué dans ledit Exemple.

| EXEMPLES : | n° 10 | n° 11 | n° 12 |
|---|---|---|---|
| Essences végétales (1/3 menthol, 1/3 thymol, 1/3 eucalyptol) | 9 % | 2 % | |
| Coal-tar saponiné . . . . . . . | | 10 % | 12 % |
| Polyéthylène . . . . . . . . . | | | 90 % |
| E.V.A.   . . . . . . . . . . | 91 % | 88 % | |

Les compositions des Exemples 10, 11 et 12 sont mises en forme par moulage à une température de l'ordre de 95°C.

II - DISPOSITIFS DE TRAITEMENT ANTICHUTE OU FAVORISANT LA REPOUSSE DES CHEVEUX :

Les compositions suivantes ont été préparées en parties en poids :

| EXEMPLES | | n° 13 | n° 14 | n° 15 |
|---|---|---|---|---|
| A | Vitamine A (acétate) . . . | 3 % | | |
| | Vitamine E . . . . . . . . | 2 % | | |
| | Vitamine F . . . . . . . . | 12 % | | |
| | Biotine (Vit H) . . . . . . | | | 6 % |
| | Cystine . . . . . . . . . | | | 10 % |
| | Essences végétales . . . . | 2 % | 1 % | |
| | Chlorhydrate de thiamine . | | 0,5 % | |
| | Nicotinamide (Vit PP) . . | | 0,9 % | |
| | Pantothénol .. . . . . . . | | 0,7 % | |
| | Vitamine C . . . . . . . . | | 0,1 % | |
| B | Glycérol . . . . . . . . . | | 10 % | |
| C | Huile de soja époxidée . . | 2 % | 2 % | 3 % |
| | Stéarate de calcium . . . | | 3 % | 2% |
| | Dioctyladipate (DOA) . . . | | | 25 % |
| | Dibutylphtalate . . . . . | 15 % | 24 % | |
| | Colorant . . . . . . . . | 0,3 % | 0,3 % | |
| D | Butadiene acrylonitrile . . | 2 % | 5 % | |
| | Chlorure de polyvinyle . . | 59,5 % | 52,5 % | 54 % |

22

Pour la mise en forme des produits à partir des compositions ci-dessus, on solubilise le mélange A dans le glycérol, dans le cas de l'Exemple 14 et l'ensemble est mélangé à la composition C tandis que la composition A seule est mélangée à ladite composition C dans le cas des Exemples 13 et 15. L'ensemble est avantageusement microsolubilisé avant d'être mélangé à D.

Pour les Exemples 13 et 14 on utilise comme solvant des agents actifs et potentialisateur de leur relargage, respectivement, le glycérol et la Vit. F.

La fabrication a lieu comme indiqué ci-dessus pour les exemples 1 à 4.

On peut avantageusement inclure dans la substance active des agents dont le rôle est de favoriser le passage des agents actifs à travers le cuir chevelu, par exemple : alkyl sulfoxides (DMSO), diméthylacétamide, diméthylformamide, pyrrolidone et dérivés (Azone ND), N-N-diéthyl-m-toluamide.

On constate de façon surprenante que dans les conditions de l'invention le taux de dégradation des vitamines est très faible durant la fabrication et qu'ensuite celles-ci bénéficient d'une protection de part leur inclusion dans la matrice les protégeant de la lumière et de l'humidité (la surface du polymère est hydrophobe) ainsi que de variations de pH(le pH dans la matrice doit être compris entre environ 3 et 6).

EXEMPLES :

| | n° 16 | n° 17 | n° 18 | n° 19 |
|---|---|---|---|---|

On prépare les compositions suivantes, en parties en poids.

| | n° 16 | n° 17 | n° 18 | n° 19 |
|---|---|---|---|---|
| Huile végétale (contenant de l'acétate de cyprostérone en solution à saturation) | 25 % | | | |
| Acide nicotinique . . . | 3 % | | | |
| (solubilisé dans du propylèneglycol) . . . . . . . | 8 % | | | |
| TWIN 80 (ND) . . . . . | 12 % | | | |
| Huile de cade . . . . . | | 3 % | | |
| Extraits de camomille . | | 5 % | | |
| Thujaplicine . . . . . | | | 7 % | |

| | | | | |
|---|---|---|---|---|
| Essence de menthol . . | | | | 4 % |
| Chlorure de caproyle .. | | | | 5 % |
| | | | | |
| D.B.P. . . . . . . . . . | 12 % | 7 % | 25 % | 22 % |
| Huile de soja époxydée . | 3 % | 2 % | 3 % | 4 % |
| Stéarate de calcium . .. | 2 % | 2 % | 2 % | 2 % |
| | | | | |
| P.V.C. . . . . . . . . . | 58 % | 66 % | 62 % | 56 % |

Les compositions des Exemples 16 à 19 sont mises en forme en effectuant un moulage conduit à une température de 95°C.

EXEMPLE N° 20

Des compositions comparables à celles de l'Exemple 17 ont été préparées en utilisant comme agents actifs des phospholipides, lipoaminoacides ou polyols d'ester d'acide gras et lesdites compositions ont été mises en forme comme décrit dans ledit exemple.

EXEMPLE N° 21

Des compositions comparables à celles de l'Exemple n° 18 ont été préparées en utilisant des extraits liposolubles de capucine, ortie blanche, quinquina, henné, lait d'avoine ou des extraits liposolubles d'origine animale : tissulaire, sanguin, placentaire, amniotique, chignon de cheval, huiles de poisson, lecithine et lesdites compositions ont ensuite été mises en forme comme indiqué dans l'Exemple 18.

EXEMPLE N° 22

Des compositions comparables à celles des Exemples 1 et 15 ont été préparées en utilisant à titre d'agent actif la DL-méthionine, la S-carboxyméthylcystéine ou l'acide gluconique. et lesdites compositions ont ensuite été mises en forme comme indiqué dans ces Exemples.

EXEMPLE N° 23

On a réalisé des compositions comparables à celles des Exemples 13 et 17 en utilisant à titre d'agent actif pour le conditionnement de la chevelure des oligomères de silicones, des esters et polyesters d'acide gras, des alcools à poids moléculaire élevé et leurs dérivés, des dérivés d'ammonium quaternaire et des dérivés de la bétaïne et lesdites compositions ont ensuite été mises en forme comme décrit en référence à ces Exemples.

On décrit maintenant, dans ce qui suit, les résultats d'essais destinés à illustrer le procédé d'utilisation des dispositifs selon l'invention.

Essais de traitements comparatifs.

Essai N° 1 - L'essai a pour but de comparer l'efficacité clinique d'un shampooing du commerce contenant du coal-tar et d'une brosse au coal-tar selon la présente invention. Les brosses avec lesquelles l'essai est réalisé sont de forme carrée, de 8,7 cm de côté, entièrement composées de la matrice polymère macromoléculaire contenant la substance active et présentant sur l'une des faces environ 225 picots de 0,75 cm de longueur.

Il a porté sur 60 personnes (3 groupes de 20) souffrant de psoriasis du cuir chevelu depuis plus de 3 mois, la durée moyenne de l'affection étant de 1,1 an ($\pm$ 0,15). Trois groupes ont été sélectionnés d'une façon aussi homogène que possible du point de vue de la durée et de l'étendue de l'affection du cuir chevelu. Le groupe A devait se laver les cheveux une fois tous les trois jours avec un shampooing placebo et se brosser les cheveux de façon biquotidienne avec la brosse active. Le groupe B devait suivre le protocole suivant : lavage des cheveux une fois tous les trois jours avec le shampooing au coal-tar et brossage biquotidien avec une brosse placebo.

Le groupe C n'utilisait que les placebo selon le même protocole.

Durant l'essai deux paramètres ont été suivis : le degré de desquamation et l'état graisseux du cuir chevelu, ceci au 30ème et au 60ème jour du traitement. Aucun autre traitement du cuir chevelu n'a été administré durant l'expérience.

Les résultats sont présentés dans le tableau ci-après :

|  | Groupe A | | Groupe B | | Groupe C | |
|---|---|---|---|---|---|---|
|  | 30 j | 60 j | 30 j | 60 j | 30 j | 60 j |
| **Etat graisseux** | | | | | | |
| mieux | 3 | 10 | 4 | 6 | 3 | 1 |
| pire | 2 | 0 | 1 | 0 | 7 | 11 |
| pas de changement | 15 | 10 | 15 | 14 | 10 | 8 |
| **Desquamation** | | | | | | |
| mieux | 9 | 13 | 13 | 12 | 2 | 0 |
| pire | 4 | 3 | 3 | 3 | 8 | 4 |
| pas de changement | 7 | 4 | 4 | 5 | 10 | 16 |

### Essai N° 2 -

Selon un protocole identique à celui de l'essai précédent, on a comparé l'action d'une brosse au pyrithione-zinc à un shampooing au pyrithione-zinc du commerce. Le paramètre étudié est la présence de pellicules.

Les résultats sont présentés dans le tableau ci-après :

|  | shampooing actif Brosse placebo | | shampooing placebo Brosse active | | shampooing et brosse placebo | |
|---|---|---|---|---|---|---|
|  | 30 j | 60 j | 30 j | 60 j | 30 j | 60 j |
| Pas de pellicules | 9 | 15 | 4 | 14 | 2 | 4 |
| mieux | 3 | 4 | 8 | 4 | 3 | 2 |
| pire | 1 | 0 | 0 | 0 | 7 | 6 |
| pas de changement | 7 | 1 | 8 | 2 | 8 | 8 |

26

Essai N° 3 - TRAITEMENT ANTI-PELLICULAIRE

L'essai avait pour but de comparer l'efficacité d'un shampooing du commerce contenant du pyrion disulfide et d'une brosse selon l'exemple 1 dans laquelle la pyrithione zinc est remplacée par du pyrion disulfide. La brosse est de forme carrée (8,7 cm de côté), entièrement composée de la matrice polymère active, présentant sur une des faces 225 picots de 0,75 cm de long.

Il a porté sur 60 personnes (3 groupes de 20, homogènes au niveau pathologique) souffrant d'hyperséborrhée du cuir chevelu et de pellicules depuis plus de 6 semaines. Le groupe A se lavait les cheveux tous les 3 jours avec un shampooing placebo et se coiffait biquotidiennement avec la brosse active. Le groupe B utilisait tous les 3 jours un shampooing au pyrion disulfide et se coiffait biquotidiennement avec une brosse placebo. Le groupe C n'utilisait que des placebo selon le même protocole. Le paramètre contrôlé est la présence de pellicules :

|  | Groupe A | | Groupe B | | Groupe C | |
|---|---|---|---|---|---|---|
|  | 30j. | 60j. | 30 j. | 60j. | 30j. | 60 j. |
| Pas (absence) de pellicules | 9 | 16 | 4 | 11 | 4 | 3 |
| mieux | 10 | 3 | 7 | 6 | 2 | 1 |
| pire | 0 | 0 | 1 | 0 | 5 | 7 |
| pas de changement | 1 | 1 | 8 | 3 | 9 | 9 |

Essai N° 4 - TRAITEMENT DES RIDES.

L'essai avait pour but d'apprécier l'activité du dispositif décrit dans l'Exemple n° 13 pour le traitement des rides du visage. La matrice active avait la forme d'un rouleau tournant sur un axe. Le protocole consistait à masser les zones ridées deux fois par jour en roulant le dispositif. L'essai a été réalisé sur 10 femmes au niveau des pommettes durant 4 semaines. Les résultats, obtenus par analyse de macrophoto de contre-empreinte du relief cutané, ont montré une réduction moyenne des rides visibles de 29 % pouvant aller jusqu'à 38 % dans certains cas.

Essai N° 5 - TRAITEMENT DE LA CHUTE DES CHEVEUX L'essai avait pour but d'apprécier l'activité du dispositif selon l'Exemple 17 dans le traitement des alopécies du cuir chevelu. Il a été réalisé sur un groupe de 60 malades (dont 20 du sexe féminin), tous suivis depuis plus d'un an et qui, après avoir tiré un certain bienêtre des traitements classiques, avaient vu leur état se stabiliser avec persistance de zones alopéciques importantes.

Le dispositif a été utilisé par la moitié des malades groupe A, répartis en deux lots homogènes aux niveaux pathologies et zones, à raison d'un minimum de deux brossages du cuir chevelu et des cheveux par jour durant 4 mois. Le groupe B a utilisé une lotion contenant les mêmes agents actifs appliquée un soir sur deux durant 40 jours puis appliquée à raison de deux fois par semaine durant 80 jours.

Chez les femmes toutes les alopécies étaient de types seborrhéiques avec éclaircissement net au niveau vortex et globes temporo-frontaux. Chez les hommes, il s'agissait dans tous les cas choisis d'alopécie avec éclaircissement du toupet et début d'alopécie au niveau de la tonsure :

Résultats :

| | |
|---|---|
| Réponse totale de cheveux normaux | : A |
| Réponse partielle de cheveux normaux (diminution des zones claires) | : B |
| Réponse partielle de cheveux fins | : C |
| Pas de résultat visible (échec) | : D |

| Lot A | A | B | C | D |
|---|---|---|---|---|
| Hommes | 0 | 2 | 15 | 3 |
| Femmes | 3 | 3 | 4 | 0 |
| Lot B | | | | |
| Hommes | 0 | 0 | 12 | 8 |
| Femmes | 1 | 2 | 5 | 2 |

28

## REVENDICATIONS

1. Dispositif pour le traitement de l'épithélium humain externe en particulier de zones sensibles de la peau, comme celles du visage, du cuir chevelu et/ou des cheveux, à l'aide d'un objet destiné à venir au contact de l'épithélium et/ou de ses phanères pour y déposer une substance active, caractérisé en ce que ledit objet (10, 20...) conformé en tant qu'article de massage de la peau ou en tant qu'instrument de coiffure est, au moins dans ses parties destinées à entrer en contact avec la peau et/ou le cuir chevelu et/ou les cheveux, constitué en une matrice macromoléculaire rigide incorporant au moins une substance active choisie pour son action thérapeutique et/ou cosmétologique et pour être propre à être relarguée vers la surface externe de ladite matrice, d'où elle est éliminée par massage et/ou contact et/ou frottement avec la peau, le cuir chevelu et/ou les cheveux.

2. Dispositif selon la revendication 1, caractérisé en ce que l'article de massage, ou l'instrument de coiffure, conformé suivant un peigne, une brosse, une barrette, un bigoudi, un rouleau, une broche, une pince, ou analogue comprend une armature qui en renforce la rigidité (13,18,23) sur laquelle est fixée la matrice macromoléculaire incorporant la ou les substances actives.

3. Dispositif selon l'une des revendications 1 ou 2, caractérisé en ce que les zones destinées à entrer en contact avec la peau et/ou le cuir chevelu et/ou les cheveux (15,22) et qui sont réalisées en ladite matrice macromoléculaire armée ou non armée forment un ensemble fixé de manière amovible ou non sur un manche, poignée de préhension ou analogue (24) en un matériau différent de celui de la matrice macromoléculaire

4. Dispositif selon l'une quelconque des revendications précédentes, caractérisé en ce que la substance active est choisie pour contenir un ou des agents à action thérapeutique et/ou cosmétologique actifs dans le traitement de la peau, en particulier le traitement des phénomènes liés au vieillissement, comme les rides du visage.

5. Dispositif selon l'une quelconque des revendications 1 à 3, caractérisé en ce que la substance active contient un ou des agents actifs dans le traitement de l'hyper-séborrhée, du psoriasis, de la chute des cheveux, de la disparition des pellicules, ou de l'aide à la repousse des cheveux.

6. Dispositif selon l'une quelconque des revendications 1 à 3, caractérisé en ce que la ou les substances actives est(sont) choisie(s) parmi celles contenant un ou des agents à action cosmétique pour faciliter le coiffage, le démêlage, le conditionnement, le nettoyage, la modification de la texture, de la couleur, et/ou de l'aspect des cheveux.

7. Dispositif selon l'une quelconque des revendications précédentes, caractérisé en ce que la ou les substances actives a(ont) un rôle de plastifiant de la matrice macromoléculaire.

8. Dispositif selon l'une quelconque des revendications précédentes, caractérisé en ce que la substance active contient une ou des vitamines et/ou des acides gras insaturés.

9. Dispositif selon l'une quelconque des revendications précédentes, caractérisé en ce que la substance active contient de 1 à 25 % d'extraits végétaux et/ou animaux et/ou d'essences végétales.

10. Dispositif selon l'une quelconque des revendications précédentes, caractérisé en ce que la substance active contient un ou des ingrédients favorisant le passage transcutané des agents actifs.

11. Dispositif selon l'une quelconque des revendications précédentes, caractérisé en ce que la matrice contient de 3 à 25 % de pyrithione ou de ses dérivés en tant qu'agent actif.

020 1387

12 . Dispositif selon l'une quelconque des revendications 1 à 10, caractérisé en ce que la matrice contient de 1 à 25 % de coal-tar ou de ses dérivés en tant qu'agent actif.

13. Dispositif selon l'une quelconque des revendications 1 à 10, caractérisé en ce que la substance active contient un agent absorbant les graisses comme de la poudre d'iris ou de lycopode, un agent détergent ou des oligomères de silicone, des dérivés d'acides gras, ou des composés d'ammonium quaternaires.

14. Dispositif selon l'une quelconque des revendications 1 à 13, caractérisé en ce que la matrice est à base de PVC plastifié et/ou de polyéthylène et/ou de polyuréthane moulé, extrudé ou coulé.

15. Dispositif selon l'une quelconque des revendications précédentes, caractérisé en ce que la quantité de plastifiant que comprend la matrice est comprise entre 0 et 25 % en poids de la matrice, le ou les polymères représentant environ 45 à 90 % en poids et la substance active de 2 à 25 % en poids.

16. Procédé de fabrication d'un dispositif selon l'une quelconque des revendications précédentes, caractérisé en ce que l'on prépare une poudre sèche par mélange à une température d'environ 60° d'un ou des polymères constitutifs de la matrice avec un ou des agents actifs préalablement micronisé(s) lorsqu'il(s) est(sont) sous forme solide ou microsolubilisé(s) dans un mélange de plastifiant et d'adjuvants, en ce qu'on laisse refroidir ladite poudre à laquelle sont alors ajoutés des ingrédients, comme des parfums ou analogues, et en ce que le dispositif est mis en forme par moulage ou extrusion à une température comprise entre 80 et 140°C dans un moule de forme appropriée.

17. Procédé de fabrication selon la revendication 16, caractérisé en ce qu'on solubilise le(les) agent(s) actif(s) dans un solvant organique avant de le mélanger au(x) plastifiant(s) et/ou aux additifs et/ou à la matrice macromoléculaire.

18. Procédé d'utilisation d'un dispositif selon l'une quelconque des revendications 1 à 16, pour l'application sur l'épithélium externe humain, en particulier les zones sensibles de la peau, comme celle du visage, le cuir chevelu et/ou les cheveux, d'un ou de principe(s) actif(s) contre le vieillissement et/ou les états pathologiques dudit épithélium et/ou pour le conditionnement de ses phanères, caractérisé en ce que ledit dépôt résulte des massages, contacts et/ou frottements répétés, mais espacés dans le temps, sur et/ou avec les zones à traiter d'un dispositif constitué par une matrice macromoléculaire dans laquelle est incorporée une substance active propre à être relarguée vers la surface et dont elle est éliminée par lesdits massages, contacts ou frottements.

## FIG. 1

## FIG. 2

## FIG. 3

Office européen
des brevets

**RAPPORT DE RECHERCHE EUROPEENNE**

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| X,D | GB-A- 632 544 (R. SAMUEL & M. WOTZILKA) * En entier * | 1-6,18 | A 61 K 7/00 A 45 D 24/00 |
| X,P | DE-A-3 426 122 (HENKEL) * En entier * | 1-6,8, 9,13, 18 | |
| X,D | EP-A-0 122 605 (WELLCOME FOUNDATION) * En entier * | 1-3,14 | |
| Y | US-A-3 682 848 (R.C. VIRNELSON) * En entier * | 1-6,16 ,18 | |
| Y,D | GB-A- 413 767 (A.A. BROOKS) * En entier * | 1-6,16 ,18 | **DOMAINES TECHNIQUES RECHERCHES (Int. Cl.4)** A 61 K A 45 D |
| Y,D | FR-A-2 385 392 (PROCTER & GAMBLE) * En entier * | 1-6,8, 9,16, 18 | |
| Y | GB-A- 332 632 (E.L. LEE) * En entier * | 1-6,8, 9,16, 18 | |

-/-

Le présent rapport de recherche a été établi pour toutes les revendications

| Lieu de la recherche | Date d'achèvement de la recherche | Examinateur |
|---|---|---|
| LA HAYE | 10-07-1986 | FISCHER J.P. |

CATEGORIE DES DOCUMENTS CITES

X : particulièrement pertinent à lui seul
Y : particulièrement pertinent en combinaison avec un autre document de la même catégorie
A : a- ∼plan technologique
O : d: non-écrite
P : d∪ ⊂alaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

OEB Form 1503 03 82

**Office européen des brevets**

**RAPPORT DE RECHERCHE EUROPEENNE**

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| A,D | US-A-2 154 822 (S. QUISLING) <br> * En entier * | 1-18 | |
| | ----- | | |

DOMAINES TECHNIQUES RECHERCHES (Int. Cl.4)

Le présent rapport de recherche a été établi pour toutes les revendications

| Lieu de la recherche | Date d'achèvement de la recherche | Examinateur |
|---|---|---|
| LA HAYE | 10-07-1986 | FISCHER J.P. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

OEB Form 1503.03.82